# FASCICULE DE BREVET EUROPEEN

(11) **EP 1 178 799 B1**
(45) Date de publication et mention de la délivrance du brevet: **27.08.2003**
(21) Numéro de dépôt: 00929640.1
(22) Date de dépôt: 19.05.2000
(51) Int. Cl.: A61K 31/44, A61K 9/50

(54) **MICROGRANULES GASTROPROTEGES, PROCEDE D'OBTENTION ET PREPARATIONS PHARMACEUTIQUES**
GEGEN MAGENSAFT GESCHÜTZTE OMEPRAZOL-MIKROGRANÜLEN, HERSTELLUNGSVERFAHREN UND PHARMAZEUTISCHE ZUBEREITUNGEN
MICROGRANULES INSOLUBLE IN GASTRIC FLUID, METHOD FOR OBTAINING SAME AND PHARMACEUTICAL PREPARATIONS

(30) Priorité: 21.05.1999 FR 9906479
(43) Date de publication de la demande: 13.02.2002
(73) Titulaire: LABORATOIRES DES PRODUITS ETHIQUES ETHYPHARM, 78550 Houdan (FR)
(72) Inventeur: CRIERE, Bruno, F-27930 Gravigny (FR); SUPLIE, Pascal, F-27400 Montaure (FR); OURY, Pascal, F-78150 Le Chesnay (FR)
(74) Mandataire: Warcoin, Jacques
(86) Numéro de dépôt international: FR0001367
(87) Numéro de publication internationale: WO00071121

(56) Documents cités:
- WO-A-93/25204
- WO-A-96/01624
- WO-A-98/52564
- WO-A-99/38511
- CHEMICAL ABSTRACTS, vol. 130, no. 14, 5 avril 1999 (1999-04-05) Columbus, Ohio, US; abstract no. 187185, XP002128930 & WO 99 06032 A (INTEXIM S.A.,SPAIN) 11 février 1999 (1999-02-11)

## Description

La présente invention concerne une formulation galénique d'un inhibiteur de la pompe à protons gastrique, à l'exception de l'Oméprazole. Cette formulation est sous la forme de microgranules gastroprotégés ayant une stabilité dans le temps améliorée.

La présente invention s'étend en outre au procédé de fabrication desdits microgranules, et aux préparations pharmaceutiques les contenant.

Les inhibiteurs de la pompe à protons gastrique entrant dans le cadre de la présente invention sont des dérivés du benzimidazole ou du thiénoimidazole, à l'exception de l'Oméprazole, ainsi que leurs sels pharmaceutiquement acceptables.

Parmi les inhibiteurs de la pompe à protons entrant dans le cadre de la présente invention, figurent notamment le Lansoprazole, le Pantoprazole, le Perprazole, le Pariprazole, le Leminoprazole, le Timoprazole et leurs sels pharmaceutiquement acceptables.

Les inhibiteurs de la pompe à protons sont connus pour le traitement et la prévention des maladies relatives à la sécrétion excessive d'acide gastrique, comme l'oesophagite, la gastrite, la duodénite, l'ulcère gastrique et l'ulcère duodénal.

Ces composés peuvent également être utilisés chez les patients qui suivent une thérapie contre le SIDA, et chez les patients souffrant de reflux gastro-oesophagique ou de gastrinome.

Enfin, ces composés sont utiles pour le traitement du psoriasis et des infections causées par Hélicobacter.

Les dérivés du benzimidazole de l'invention sont des composés susceptibles de dégradation en milieu acide ou neutre, si bien qu'une formulation les contenant doit être :
- entérique pour que le principe actif atteigne l'intestin grêle, et
- protégée contre la chaleur, l'humidité, les solvants organiques, et la lumière à un moindre degré.

La présente invention concerne une nouvelle formulation gastroprotégée d'un inhibiteur de la pompe à protons contenant au moins deux substances hydrophobes, dont la fonction est d'améliorer la stabilité de la formulation au stockage.

Il existe déjà dans l'art antérieur des formulations contenant un dérivé du benzimidazole et une substance hydrophobe, mais cette substance n'est pas nommément utilisée pour augmenter la stabilité de la formulation. En outre, ces formulations contiennent des composés alcalins et/ou des tensio-actifs ioniques.

WO 96/01624 décrit des comprimés de microgranules entériques contenant un inhibiteur de la pompe à protons. L'objectif de l'invention décrite est de préparer des microgranules compressibles sans modifier les propriétés de leur couche entérique. Pour obtenir les propriétés mécaniques souhaitées, la couche entérique doit contenir des plastifiants, comme les polysorbates, les PEG et l'alcool cétylique. La couche entérique est par exemple constituée de 74-80% d'un copolymère méthacrylique, 16-23% de triéthylcitrate et 1-3% d'un mélange monoglycérides/diglycérides. WO 96/01624 décrit l'utilisation d'un plastifiant particulier dans la couche entérique pour améliorer la compressibilité des granules. En outre, les compositions décrites contiennent un tensio-actif ionique, comme le lauryl sulfate de sodium ou un sel alcalin, comme le phosphate de calcium.

WO 97/12581 décrit des granules d'Oméprazole stables dépourvus de tout composé alcalinisant. De façon classique, la couche entérique comprend un plastifiant comme le triéthylcitrate. Les granules peuvent contenir des lubrifiants, présentant des propriétés hydrophobes. Par ailleurs, un tensio-actif ionique comme le lauryl sulfate de sodium, ou la crospovidone qui est dotée d'un caractère alcalin sont associés au principe actif.

WO 98/19668 décrit des granules d'Oméprazole dont la stabilité est améliorée en intercalant une couche barrière entre l'enrobage entérique et le principe actif. Cette couche barrière sert à protéger le principe actif de l'humidité ambiante et du polymère entérique doté d'un caractère acide. Ce document ne suggère pas d'utiliser une substance hydrophobe dans la couche barrière, mais elle peut contenir de la Siméthicone® en une proportion massique de 0,4% par rapport au poids du principe actif. Le Myvacet® est employé comme plastifiant dans la couche entérique. Les granules sont constitués d'un coeur alcalin pouvant contenir des hydroxydes ou des oxydes de magnésium, calcium, aluminium, du trisodium phosphate ou du trisilcate de magnésium.

Les documents de l'art antérieur qui portent sur des formulations orales solides d'inhibiteurs de la pompe à protons dont on cherche à augmenter la stabilité décrivent des formulations qui contiennent des substances hydrophobes, mais ces documents n'enseignent pas que les substances hydrophobes sont utiles pour augmenter la stabilité au stockage. Au contraire, dans ces documents, les substances hydrophobes sont utilisées de façon classique comme plastifiant dans la couche entérique (WO 96/01624, WO 97/12581, WO 98/19668), comme lubrifiant (WO 97/12581) ou comme liant (WO 98/19668). Par ailleurs, les compositions qu'ils décrivent contiennent des composés alcalins ou des tensio-actifs ioniques.

Afin d'améliorer la durée de stabilité au stockage de formulations gastroprotégées contenant un inhibiteur de la pompe à protons, on a proposé dans l'art antérieur d'introduire une substance alcaline et une substance hydrophobe dans la formulation.

WO 98/52564 décrit des granules de benzimidazole comprenant un noyau inerte enrobé d'une couche contenant le principe actif associé à une substance alcaline, d'une couche barrière constituée d'une substance hydrophobe, et d'une couche entérique. La substance hydrophobe est un polyalkylsiloxane, une huile minérale, le myristate d'isopropyle, l'acide stéarique ou l'alcool cétylique. La substance alcaline est par exemple l'ammoniaque, l'hydroxyde d'ammonium ou le carbonate d'ammonium.

WO 98/52564 propose d'améliorer la stabilité de granules de benzimidazole en intercalant un film hydrophobe entre le principe actif et l'enrobage entérique et en associant une substance alcaline au principe actif.

Il existe également dans l'art antérieur deux documents concernant des formulations d'un inhibiteur de la pompe à protons qui ne sont pas gastroprotégées, qui ne sont pas sous forme de microgranules et qui contiennent des substances hydrophobes.

WO 96/31213 porte sur une formulation orale pâteuse d'un inhibiteur de la pompe à protons pour un usage vétérinaire ou pour des personnes ayant des difficultés à avaler. Cette formulation est stable au stockage de longue durée. Elle contient un véhicule liquide huileux hydrophobe et un agent épaississant hydrophobe. Le véhicule huileux est par exemple le Miglyol 810®. L'agent épaississant est l'alcool cétostéarylique, la paraffine ou l'huile de ricin hydrogénée. La formulation contient également des agents alcalinisants, comme le sorbate de potassium ou la triéthanolamine. L'enseignement de ce document est spécifique d'une formulation semi-solide.

EP 769 938 décrit des capsules molles à libération prolongée contenant des substances actives instables à l'humidité, à l'oxydation et au fluide gastrique.

Les capsules molles classiques sont principalement constituées d'une masse de gélatine hydratée, et de nombreux additifs qui peuvent s'avérer incompatibles avec le principe actif. EP 769 938 garantit la stabilité des capsules molles contenant des principes actifs sensibles à l'humidité en isolant le principe actif de la masse gélatineuse. Les capsules molles de EP 769 938 sont constituées
- d'un coeur contenant le principe actif et 70 % de silicone, enrobé
- d'une première couche constituée de gélatine, de sorbitol et de glycérol, enrobée
- d'un film de silicone.

L'enseignement de EP 769 938 est limité à des capsules molles qui ne sont pas gastroprotégées.

Il n'existe pas dans l'art antérieur une formulation gastroprotégée de microgranules contenant un dérivé du benzimidazole stable au stockage, dépourvue de substances alcalines et contenant une substance hydrophobe à la fois dans la couche active et dans la couche entérique.

Le but de la présente invention est de fournir une formulation gastroprotégée de microgranules d'un inhibiteur de la pompe à protons gastrique, à l'exception de l'Oméprazole, dont la stabilité au stockage de longue durée est améliorée, et qui présente, en outre, les propriétés thérapeutiques voulues, c'est-à-dire une certaine résistante à la dissolution en milieu acide, et une solubilité rapide en milieu neutre.

La présente invention concerne une nouvelle formulation gastroprotégée d'un inhibiteur de la pompe à protons, à l'exception de l'Oméprazole contenant plusieurs substances hydrophobes choisies pour augmenter la stabilité du principe actif tout en obtenant le profil de dissolution désiré.

Les microgranules objet de la présente invention sont avantageusement dépourvus:
- de composés alcalins, i.e. dont le pH est supérieur ou égal à 7, par exemple, les bases aminées comme l'ammoniaque, la triéthanolamine ; les sels d'acides carboxyliques comme le citrate de sodium ou le sorbate de potassium ; des carbonates, phosphates, hydroxydes ou oxydes de sodium, aluminium, potassium, magnésium ou calcium ; le trisilicate de magnésium ; le tris(hydroxyméthyl) aminométhane ; les argiles naturelles comme la montmorillonite ; le glycérophosphate de sodium ; le borate de sodium ; les tampons organiques ; la crospovidone,
- d'agents tensioactifs ioniques, comme le lauryl sulfate, et
- de traces de solvants organiques.

Les microgranules selon l'invention contiennent un inhibiteur de la pompe à protons gastrique à l'exception de l'Oméprazole, et comportent chacun une couche active contenant le principe actif et une couche externe de gastroprotection. Ils sont caractérisés en ce que la couche active et la couche de gastroprotection contiennent chacune au moins une substance hydrophobe choisie pour augmenter la stabilité des microgranules au stockage. Les microgranules de l'invention sont dépourvus de tout composé alcalin et de tout tensio-actif ionique.

On choisira des substances hydrophobes qui ne réagissent pas chimiquement avec le principe actif, qui peuvent être facilement mises en oeuvre lors de la formulation et qui sont compatibles avec les excipients utilisés.

Dans le cadre de la présente invention, on entend par substance hydrophobe, toute substance permettant d'obtenir un gain de stabilité des microgranules au stockage, notamment toute substance présentant un HLB inférieur à 15, ou non hygroscopique, ou pratiquement insoluble dans l'eau, ou formant un film non perméable à la vapeur d'eau.

Dans la couche active, la substance hydrophobe représente de préférence entre 5 et 40 % en poids du principe actif. Elle est avantageusement choisie parmi les huiles siliconées.

Il peut également être inclus dans la couche active 5 à 15 %, par rapport au poids de principe actif, d'un tensio-actif non ionique choisi de préférence parmi les polysorbates (Montanox 80® ou Montane 20-60®).

La couche active comporte avantageusement un liant choisi parmi les liants pharmaceutiquement acceptables, par exemple l'hydroxypropyl méthylcellulose dont la proportion massique représente de préférence 30 à 50 % par rapport au poids de principe actif.

La couche externe de gastroprotection est avantageusement constituée d'un agent filmogène gastroprotecteur, d'une substance hydrophobe et d'un plastifiant hydrophile.

Avantageusement, la substance hydrophobe contenue dans la couche de gastroprotection est choisie parmi les cires, les huiles et leurs mélanges souvent utilisés dans l'industrie pharmaceutique, préférentiellement les glycérides, par exemple les Gélucire®, en proportion de 5 à 20 % du vernis sec de l'agent filmogène.

Le plastifiant est choisi parmi les plastifiants pharmaceutiquement acceptables, par exemple les PEG, l'alcool cétylique ou le triéthylcitrate.

Le plastifiant représente de 5 à 20 %, avantageusement 10 %, du poids de vernis sec de l'agent filmogène.

L'agent filmogène gastroprotecteur est avantageusement un copolymère d'acide méthacrylique, comme l'Eudragit L30D®, à raison de 15 à 60 % du dépôt sec de polymère par rapport à la masse de microgranules.

Afin de renforcer la résistance à l'humidité de la couche de gastroprotection, on utilise éventuellement un agent lubrifiant choisi parmi les lubrifiants pharmaceutiquement acceptables, avantageusement le talc.

La couche de gastroprotection est avantageusement constituée de 90 à 95% d'agent filmogène, et d'une quantité égale de plastifiant et de substance hydrophobe.

Selon un mode de réalisation préféré de l'invention, au moins une couche intermédiaire est intercalée entre la couche active et la couche de gastroprotection. La couche intermédiaire peut également contenir une substance hydrophobe, qui représente de préférence entre 5 et 40 % en poids du principe actif.

La couche intermédiaire peut contenir une substance diluante ou un agent d'enrobage associé à un plastifiant hydrophobe.

Selon un mode de réalisation préféré, les microgranules selon l'invention comportent :
- une couche de principe actif contenant un principe actif, un liant choisi parmi les liants pharmaceutiquement acceptables, une substance hydrophobe et un tensio-actif non ionique,
- une première couche de protection contenant une ou plusieurs substances diluantes hydrophobes pharmaceutiquement acceptables, et un liant choisi parmi les liants pharmaceutiquement acceptables,
- une deuxième couche de protection hydrophobe contenant un agent d'enrobage et un plastifiant hydrophobe,
- une couche de gastroprotection contenant un agent filmogène entérique, un plastifiant hydrophile et une substance hydrophobe.

La première couche de protection intermédiaire comporte avantageusement du mannitol (qui est non hygroscopique) en proportion massique de 100 à 300 % et, préférentiellement, 200 % du poids du principe actif.

Cette couche comporte également un liant choisi parmi les liants pharmaceutiquement acceptables, avantageusement l'hydroxypropylméthylcellulose, en proportion de 10 à 30 % et, préférentiellement, 20 % du poids de mannitol.

Eventuellement, il peut être inclus dans cette couche de protection un lubrifiant choisi parmi les lubrifiants pharmaceutiquement acceptables, en l'occurrence le talc (qui est non hygroscopique) en proportion inférieure à 100 % du poids du principe actif.

La deuxième couche de protection est constituée d'un agent d'enrobage hydrosoluble choisi parmi les agents filmogènes pharmaceutiquement acceptables, avantageusement l'hydroxypropylméthylcellulose, en proportion de 1 à 10 % préférentiellement 5 % du poids de microgranules obtenus après montage de la première couche de protection.

On utilisera avantageusement, dans la deuxième couche de protection, un plastifiant hydrophobe tel que le Myvacet® en proportion de 10 à 30 % du vernis sec de l'agent d'enrobage retenu.

La deuxième couche de protection peut contenir un agent lubrifiant choisi parmi les lubrifiants pharmaceutiquement acceptables tels que le talc, en proportion de 10 à 50 %, préférentiellement 15% en poids du vernis sec de l'agent d'enrobage retenu.

Selon un mode de réalisation préféré de la présente invention, la couche active est montée sur un noyau neutre constitué par exemple de saccharose et d'amidon, dont le diamètre est compris entre 200 et 900 microns.

Les microgranules selon l'invention ont, de préférence, une granulométrie comprise entre 0,3 et 3 mm, de préférence encore entre 0,4 et 2 mm.

Selon un mode de réalisation préféré, les microgranules de l'invention comprennent :
- 35 à 45 % de neutres,
- 15 à 25 % de mannitol,
- 5 à 15 % de principe actif,
- 8 à 15 % d'hydroxypropylméthylcellulose,
- 15 à 60 % d'un copolymère d'acide méthacrylique,
- 0,5 à 1,5 % d'une huile siliconée,
- 0,5 à 1,5 % d'un tensio-actif non ionique,
- 1 à 6 % d'un plastifiant,
- 1 à 6 % d'un glycéride,
- 1 à 2 % de talc,
les pourcentages étant exprimés en masse.

La présente invention a également pour objet un procédé de préparation des microgranules selon l'invention. Ce procédé est caractérisé en ce qu'il est réalisé en milieu aqueux, sans utilisation d'aucun solvant organique.

Les microgranules décrits dans la présente invention seront obtenus par utilisation de tout équipement adéquat pour la préparation et l'enrobage de microgranules, bien connu de l'homme du métier et, en particulier, les équipements de type turbine conventionnelle, turbine perforée ou lit d'air fluidisé.

Selon un mode de réalisation préféré, les microgranules selon l'invention sont obtenus par montage sur noyau neutre, de préférence, en lit d'air fluidisé, par pulvérisations successives :
- d'une suspension aqueuse de principe actif et d'une substance hydrophobe,
- éventuellement d'une suspension aqueuse d'une substance diluante,
- éventuellement d'une suspension aqueuse d'un agent d'enrobage et d'un plastifiant hydrophobe, et
- d'une suspension aqueuse de l'agent de gastroprotection dit également agent filmogène entérique.

Selon un mode de réalisation tout particulièrement apprécié, les microgranules selon l'invention sont montés sur noyau neutre en lit d'air fluidisé, par pulvérisations successives :
- d'une suspension aqueuse de principe actif et d'une huile siliconée,
- d'une suspension aqueuse de mannitol,
- d'une suspension aqueuse d'hydroxypropylméthylcellulose, et
- d'une suspension aqueuse de l'agent de gastroprotection.

Chaque étape de pulvérisation est avantageusement suivie d'un tamisage et d'un séchage à une température inférieure à la température de fusion de chacun des composés entrant dans la constitution des microgranules à ladite étape.

Les microgranules obtenus selon ce procédé contiennent avantageusement moins de 1,5 %, de préférence 0,5 % en poids d'eau.

La présente invention a enfin pour objet les préparations pharmaceutiques contenant les microgranules selon l'invention susceptibles d'être obtenus par le procédé décrit précédemment, ces préparations seront avantageusement sous forme de gélules contenant 5 à 60 mg environ de principe actif.

D'autres caractéristiques et avantages de la présente invention apparaîtront à la lumière de l'exemple ci-après.

### EXEMPLE

On prépare des microgranules dans un appareil à lit d'air fluidisé de type OHLMAN, de composition suivante :

| **Composition** | **Pourcentage massique** |
|---|---|
| Neutres | 39,3 |
| Lansoprazole | 9,2 |
| Pharmacoat 630® | 11,0 |
| Diméthicone® | 0,9 - |
| Polysorbate 80® | 0,9 |
| Mannitol | 18,3 |
| Myvacet® | 0,9 |
| Talc | 1,4 |
| Eudragit L30D® | 15,0 |
| Triéthylcitrate | 1,5 |
| Gélucire 50/13® | 1,5 |

### a) Montage du principe

L'eau purifiée est mise sous agitation et on ajoute successivement le Pharmacoat 603® (fabriqué par SEPPIC), le Polysorbate 80® (fabriqué par SEPPIC), le Diméthicone® (fabriqué par LAMBERT et RIVIERE) et le principe actif.

L'agitation de la suspension est maintenue pendant tout le montage des noyaux neutres placés dans le lit d'air fluidisé.

Les neutres enrobés sont ensuite tamisés, et séchés pendant quatre heures à 50°C environ.

### b) Prémontage Pharmacoat®/Mannitol

On prépare une suspension de prémontage constituée de 4 % en poids de Pharmacoat 603®, 20 % en poids de Mannitol 25® (tous deux fabriqués par ROQUETTE) et 76 % d'eau purifiée.

Les neutres enrobés et séchés, obtenus précédemment sont pulvérisés avec cette suspension de prémontage.

Les neutres prémontés sont ensuite tamisés, puis séchés pendant une à quatre heures à 50°C environ.

### c) Prémontage Pharmacoat®/Myvacet®

Cette étape de prémontage est effectuée dans les mêmes conditions que l'étape de prémontage Pharmacoat®/Mannitol.

Au cours des étapes a), b) et c), la température des granules est maintenue entre 26 et 28°C pendant la pulvérisation de la suspension.

### d) Enrobage Eudragit L30D®/Gelucire®

On prépare une suspension aqueuse d'enrobage contenant l'Eudragit L30D®, le triéthyl citrate et le Gélucire 50/13® en ajoutant le Gélucire® (fabriqué par GATTE FOSSE) fondu à 50°C.

Les microgranules enrobés sont ensuite tamisés et séchés à environ 45°C pendant quatre heures, puis lubrifiés avec du talc.

Les pertes à dessication des microgranules sont de l'ordre de 0,5 à 1 % après quinze minutes à 95°C, à la fin de chacune des étapes a) à d).

Les microgranules obtenus ont les propriétés suivantes :

| | |
|---|---|
| Teneur (mg/g) | 91,7 |
| Essai de gastrorésistance (% massique) | |
| au bout de 2 h à pH 1,2 | 4,14 |
| puis pendant 30 min à pH 6,8 | 82,70 |

Conformément à la Pharmacopée Européenne, les essais de dissolution in vitro sont réalisés avec un appareil à palette tournant à une vitesse de 100 tours/minute, dans 750 ml d'eau à 37°C ± 0,5°C et pH = 1,2, auxquels on ajoute au bout de deux heures, 250 ml d'une solution aqueuse de Na₃PO₄ à pH = 12,5 pour obtenir 1 I d'une solution à pH = 6,8.

## Revendications

1. Microgranules contenant un inhibiteur de la pompe à protons gastrique à l'exception de l'Oméprazole, comportant chacun une couche active contenant le principe actif et une couche externe de gastroprotection, **caractérisés en ce que** la couche active et la couche de gastroprotection contiennent chacune au moins une substance hydrophobe choisie pour augmenter la stabilité des microgranules au stockage, et **caractérisés en ce que** les microgranules sont dépourvus de tout composé alcalin et de tout tensio-actif ionique.

2. Microgranules selon la revendication 1, **caractérisés en ce** la substance hydrophobe contenue dans la couche active représente 5 à 40 % en poids du principe actif.

3. Microgranules selon l'une des revendications 1 ou 2, **caractérisés en ce que** la substance hydrophobe contenue dans la couche active est choisie parmi les huiles siliconées.

4. Microgranules selon l'une des revendications 1 à 3, **caractérisés en ce que** la couche active contient 5 à 15% par rapport au poids de principe actif d'un tensio-actif non ionique, par exemple un polysorbate.

5. Microgranules selon l'une des revendications 1 à 4, céractérisés en ce que la couche active contient un liant comme l'hydroxypropylméthylcellulose.

6. Microgranules selon l'une des revendications précédentes, **caractérisés en ce que** la couche de gastroprotection est constituée de 90 à 95% d'agent filmogène, et d'une quantité égale de plastifiant et de substance hydrophobe.

7. Microgranules selon l'une des revendications précédentes, **caractérisés en ce que** la substance hydrophobe contenue dans la couche de gastroprotection est choisie parmi les glycérides.

8. Microgranules selon l'une des revendications 6 ou 7, **caractérisés en ce que** le plastifiant représente 5 à 20% en poids du vernis sec de l'agent filmogène.

9. Microgranules selon l'une des revendications 6 à 8, **caractérisés en ce que** l'agent filmogène représentant de 15 à 60 % du dépôt sec de polymère par rapport à la masse de microgranules.

10. Microgranules selon l'une des revendications 6 à 9, **caractérisés en ce que** l'agent filmogène est un copolymère d'acide méthacrylique, comme l'Eudragit L30D®.

11. Microgranules selon l'une des revendications précédentes, **caractérisés en ce qu'**au moins une couche intermédiaire est intercalée entre la couche active et la couche de gastroprotection.

12. Microgranules selon la revendication 11, **caractérisés en ce que** chaque microgranule contient
• une couche de principe actif contenant un principe actif, un liant choisi parmi les liants pharmaceutiquement acceptables, une substance hydrophobe et un tensio-actif non ionique,
• une première couche de protection contenant une ou plusieurs substances diluantes hydrophobes pharmaceutiquement acceptables et un liant,
• une deuxième couche de protection hydrophobe contenant un agent d'enrobage et un plastifiant hydrophobe,
• une couche de gastroprotection contenant un agent filmogène entérique, un plastifiant hydrophile et une substance hydrophobe.

13. Microgranules selon la revendication 12, **caractérisés en ce que** la première couche de protection comporte du mannitol comme substance diluante.

14. Microgranules selon l'une des revendications 12 ou 13, **caractérisé en ce que** la deuxième couche de protection est constituée d'un agent d'enrobage hydrosoluble comme l'hydroxypropylméthylcellulose et d'un plastifiant hydrophobe comme le Myvacet®.

15. Microgranules selon l'une des revendications précédentes, **caractérisés en ce que** la couche active est montée sur un noyau neutre, et que la granulométrie des microgranules est comprise entre 0,3 et 3 mm.

16. Microgranules selon l'une des revendications précédentes, **caractérisés en ce qu'**ils comprennent :
- 35 à 45 % de neutres,
- 15 à 25 % de mannitol,
- 5 à 15 % de principe actif,
- 8 à 15 % d'hydroxypropylméthylcellulose,
- 15 à 60 % d'un copolymère d'acide méthacrylique,
- 0,5 à 1,5 % d'une huile siliconée,
- 0,5 à 1,5 % d'un tensio-actif non ionique,
- 1 à 6 % d'un plastifiant,
- 1 à 6 % d'un glycéride,
- 1 à 2 % de talc,
les pourcentages étant exprimés en masse.

17. Procédé de préparation des microgranules selon l'une des revendications précédentes, **caractérisé en ce qu'**il est réalisé en milieu aqueux.

18. Procédé selon la revendication 17, **caractérisé en ce qu'**on procède à un montage en lit d'air fluidisé.

19. Préparations pharmaceutiques contenant les microgranules selon l'une des revendications 1 à 16, ou obtenus selon le procédé de l'une des revendications 17 ou 18, contenant 5 à 60 mg environ de principe actif.

## Patentansprüche

1. Mikrogranalien, enthaltend einen Inhibitor der Magen-Protonenpumpe, mit Ausnahme von Omeprazol, von denen jedes eine aktive Schicht, die den Wirkstoff enthält, und eine äußere Magenschutzschicht umfasst, **dadurch gekennzeichnet, dass** die aktive Schicht und die Magenschutzschicht jeweils mindestens eine hydrophobe Substanz enthalten, die so gewählt ist, dass sie die Stabilität der Mikrogranalien bei der Lagerung verbessert, und **dadurch gekennzeichnet, dass** die Mikrogranalien keinerlei alkalische Verbindung und keinerlei ionisches Tensid enthalten.

2. Mikrogranalien nach Anspruch 1, **dadurch gekennzeichnet, dass** die hydrophobe Substanz, die in der aktiven Schicht enthalten ist, 5 bis 40 Gew.-% des Wirkstoffs beträgt.

3. Mikrogranalien nach einem der Ansprüche 1 oder 2, **dadurch gekennzeichnet, dass** die hydrophobe Substanz, die in der aktiven Schicht enthalten ist, aus siliconhaltigen Ölen ausgewählt ist.

4. Mikrogranalien nach einem der Ansprüche 1 bis 3, **dadurch gekennzeichnet, dass** die aktive Schicht 5 bis 15 %, bezogen auf das Gewicht des Wirkstoffs, eines nicht-ionischen Tensids, z.B. eines Polysorbats, enthält.

5. Mikrogranalien nach einem der Ansprüche 1 bis 4, **dadurch gekennzeichnet, dass** die aktive Schicht ein Bindemittel, wie Hydroxypropylmethylcellulose, enthält.

6. Mikrogranalien nach einem der vorangehenden Ansprüche, **dadurch gekennzeichnet, dass** die Magenschutzschicht aus 90 bis 95 % filmbildendem Mittel und einer gleichen Menge an Weichmacher und hydrophober Substanz zusammengesetzt ist.

7. Mikrogranalien nach einem der vorangehenden Ansprüche, **dadurch gekennzeichnet, dass** die hydrophobe Substanz, die in der Magenschutzschicht enthalten ist, aus Glyceriden ausgewählt ist.

8. Mikrogranalien nach einem der Ansprüche 6 oder 7, **dadurch gekennzeichnet, dass** der Weichmacher 5 bis 20 Gew.-% des trockenen Lacks des filmbildenden Mittels ausmacht.

9. Mikrogranalien nach einem der Ansprüche 6 bis 8, **dadurch gekennzeichnet, dass** das filmbildende Mittel 16 bis 60 % des trockenen Polymerüberzugs, bezogen auf das Gewicht der Mikrogranalien, ausmacht.

10. Mikrogranalien nach einem der Ansprüche 6 bis 9, **dadurch gekennzeichnet, dass** das filmbildende Mittel ein Methacrylsäure-Copolymer, wie Eudragit L30D®, ist.

11. Mikrogranalien nach einem der vorangehenden Ansprüche, **dadurch gekennzeichnet, dass** mindestens eine Zwischenschicht zwischen der aktiven Schicht und der Magenschutzschicht eingeschoben ist.

12. Mikrogranalien nach Anspruch 11, **dadurch gekennzeichnet, dass** jede Mikrogranalie enthält:
• eine Wirkstoffschicht, die einen Wirkstoff, ein Bindemittel, das aus pharmazeutisch annehmbaren Bindemitteln ausgewählt ist, eine hydrophobe Substanz und ein nicht-ionisches Tensid enthält,
• eine erste Schutzschicht, die eine oder mehrere pharmazeutisch annehmbare hydrophobe Verdünnungsmittel und ein Bindemittel enthält,
• eine zweite hydrophobe Schutzschicht, die ein Umhüllungsmittel und einen hydrophoben Weichmacher enthält,
• eine Magenschutzschicht, die ein enterisches filmbildendes Mittel, einen hydrophilen Weichmacher und eine hydrophobe Substanz enthält.

13. Mikrogranalien nach Anspruch 12, **dadurch gekennzeichnet, dass** die erste Schutzschicht Mannit als Verdünnungsmittel umfasst.

14. Mikrogranalien nach einem der Ansprüche 12 oder 13, **dadurch gekennzeichnet, dass** die zweite Schutzschicht aus einem wasserlöslichen Umhüllungsmittel, wie Hydroxypropylmethylcellulose und einem hydrophoben Weichmacher, wie Myvacet®, zusammengesetzt ist.

15. Mikrogranalien nach einem der vorangehenden Ansprüche, **dadurch gekennzeichnet, dass** die aktive Schicht auf einen neutralen Kern aufgebracht ist und dass die Teilchengröße der Mikrogranalien zwischen 0,3 und 3 mm einschließlich liegt.

16. Mikrogranalien nach einem der vorangehenden Ansprüche, **dadurch gekennzeichnet, dass** sie umfassen:
- 35 bis 45 % neutrale Stoffe,
- 15 bis 25 % Mannit,
- 5 bis 15 % Wirkstoff,
- 8 bis 15 % Hydroxypropylmethylcellulose,
- 15 bis 60 % eines Methacrylsäure-Copolymers,
- 0,5 bis 1,5 % eines siliconhaltigen Öls,
- 0,5 bis 1,5 % eines nicht-ionischen Tensids,
- 1 bis 6 % eines Weichmachers,
- 1 bis 6 % eines Glycerids,
- 1 bis 2 % Talkum,
wobei die Prozentsätze auf Gewicht bezogen sind.

17. Verfahren zur Herstellung von Mikrogranalien nach einem der vorangehenden Ansprüche, **dadurch gekennzeichnet, dass** es in wässrigem Medium durchgeführt wird.

18. Verfahren nach Anspruch 17, **dadurch gekennzeichnet, dass** man in einer Luft-Fließbettvorrichtung verfährt.

19. Pharmazeutische Präparate, enthaltend Mikrogranalien nach einem der Ansprüche 1 bis 16 oder erhalten nach dem Verfahren von einem der Ansprüche 17 oder 18, welche etwa 5 bis 60 mg Wirkstoff enthalten.

## Claims

1. Microgranules comprising a gastric proton pump inhibitor, with the exception of omeprazole, each comprising an active layer comprising the active principle and an outer layer for insolubility in gastric fluid, **characterized in that** the active layer and the layer for insolubility in gastric fluid each comprise at least one hydrophobic substance chosen in order to increase the stability of the microgranules on storage and **characterized in that** the microgranules have no alkaline compound and no ionic surfactant.

2. Microgranules according to Claim 1, **characterized in that** the hydrophobic substance present in the active layer represents 5 to 40% by weight of the active principle.

3. Microgranules according to either of Claims 1 and 2, **characterized in that** the hydrophobic substance present in the active layer is chosen from silicone oils.

4. Microgranules according to one of Claims 1 to 3, **characterized in that** the active layer comprises 5 to 15%, with respect to the weight of active principle, of a nonionic surfactant, for example a polysorbate.

5. Microgranule according to one of Claims 1 to 4, **characterized in that** the active layer comprises a binder, such as hydroxypropylmethylcellulose.

6. Microgranules according to one of the preceding claims, **characterized in that** the layer for insolubility in gastric fluid is composed of 90 to 95% of film-forming agent and of an equal amount of plasticizer and of hydrophobic substance.

7. Microgranules according to one of the preceding claims, **characterized in that** the hydrophobic substance present in the layer for insolubility in gastric fluid is chosen from the glycerides.

8. Microgranules according to either of Claims 6 and 7, **characterized in that** the plasticizer represents 5 to 20% by weight of the dry glaze of the film-forming agent.

9. Microgranules according to one of Claims 6 to 8, **characterized in that** the film-forming agent represents from 15 to 60% of the dry polymer deposit with respect to the mass of microgranules.

10. Microgranules according to one of Claims 6 to 9, **characterized in that** the film-forming agent is a copolymer of methacrylic acid, such as Eudragit L30D®.

11. Microgranules according to one of the preceding claims, **characterized in that** at least one intermediate layer is inserted between the active layer and the layer for insolubility in gastric fluid.

12. Microgranules according to Claim 11, **characterized in that** each microgranule comprises
• a layer of active principle comprising an active principle, a binder chosen from pharmaceutically acceptable binders, a hydrophobic substance and a nonionic surfactant,
• a first protective layer comprising one or more pharmaceutically acceptable hydrophobic diluent substances and a binder,
• a second hydrophobic protective layer comprising a coating agent and a hydrophobic plasticizer,
• a layer for insolubility in gastric fluid comprising an enteric film-forming agent, a hydrophilic plasticizer and a hydrophobic substance.

13. Microgranules according to Claim 12, **characterized in that** the first protective layer comprises mannitol as diluent substance.

14. Microgranules according to either of Claims 12 and 13, **characterized in that** the second protective layer is composed of a water-soluble coating agent, such as hydroxypropylmethylcellulose, and of a hydrophobic plasticizer, such as Myvacet®.

15. Microgranules according to one of the preceding claims, **characterized in that** the active layer is applied to a neutral nucleus and **in that** the particle size of the microgranules is between 0.3 and 3 mm.

16. Microgranules according to one of the preceding claims, **characterized in that** they comprise:
- 35 to 45% of neutral materials,
- 15 to 25% of mannitol,
- 5 to 15% of active principle,
- 8 to 15% of hydroxypropylmethylcellulose,
- 15 to 60% of a copolymer of methacrylic acid,
- 0.5 to 1.5% of a silicone oil,
- 0.5 to 1.5% of a nonionic surfactant,
- 1 to 6% of a plasticizer,
- 1 to 6% of a glyceride,
- 1 to 2% of talc,
the percentages being expressed by mass.

17. Process for the preparation of the microgranules according to one of the preceding claims, **characterized in that** it is carried out in aqueous medium.

18. Process according to Claim 17, **characterized in that** application is carried out in a fluidized air bed.

19. Pharmaceutical preparations comprising the microgranule according to one of Claims 1 to 16 or obtained according to the process of either of Claims 17 and 18, comprising 5 to 60 mg approximately of active principle.
